(19) Europäisches Patentamt
European Patent Office
Office européen des brevets

(11) Veröffentlichungsnummer: **0 083 555**
**B1**

# (12) EUROPÄISCHE PATENTSCHRIFT

(45) Veröffentlichungstag der Patentschrift: **29.05.85**

(51) Int. Cl.⁴: **C 07 C 143/55**

(21) Anmeldenummer: **82810566.8**

(22) Anmeldetag: **24.12.82**

## (54) Verfahren zur Herstellung von p-Nitrotoluol-2-sulfonsäure.

(30) Priorität: **31.12.81 US 336150**

(43) Veröffentlichungstag der Anmeldung: **13.07.83 Patentblatt 83/28**

(45) Bekanntmachung des Hinweises auf die Patenterteilung: **29.05.85 Patentblatt 85/22**

(84) Benannte Vertragsstaaten: **BE CH DE FR GB IT LI NL**

(56) Entgegenhaltungen:
**EP - A - 0 018 541**
**EP - A - 0 026 154**
**CH - A - 478 772**
**DE - A - 1 643 607**

**PATENTS ABSTRACTS OF JAPAN, Band 4, 15. März 1980, Seite 65C2**

(73) Patentinhaber: **CIBA-GEIGY AG, Postfach, CH-4002 Basel (CH)**

(72) Erfinder: **Lee, John Gordon, 425, Scott Drive East, Saraland Alabama 36571 (US)**
Erfinder: **Lund, Richard Boardman, 203 Dogwood Drive, Jackson Alabama 36545 (US)**

**EP 0 083 555 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein verbessertes Verfahren zur Herstellung von p-Nitrotoluol-2-sulfonsäure (im folgenden als PNTSA bezeichnet) durch Sulfonierung einer Schmelze von p-Nitrotoluol (im folgenden als PNT bezeichnet) mit gasförmigem $SO_3$ in Gegenwart einer kleinen Menge Schwefelsäure.

Tausende von Tonnen PNTSA werden jährlich hergestellt. Ein wesentlicher Teil der insgesamt hergestellten Menge wird in Form einer wäßrigen Lösung zur Herstellung von 4,4'-Dinitrostilben-2,2'-disulfonsäure (im folgenden als DNS bezeichnet) eingesetzt, die zur Herstellung einer Vielzahl von optischen Aufhellern auf Cyanurchlorid-Basis verwendet wird, beispielsweise zur Herstellung der folgenden optischen Aufheller:

4,4'-Bis-([4-anilino-6-(N-2-hydroxyäthyl-N-methylamino)-1,3,5-triazin-2-yl]amino)-stilben-2,2'-disulfonsäure-dinatriumsalz,
4,4'-Bis-[(4-anilino-6-morpholino-1,3,5-triazin-2-yl)-amino]-stilben-2,2'-disulfonsäure-dinatriumsalz,
4,4'-Bis-([4-anilino-6-(bis-(2-hydroxyäthyl)-amino)-1,3,5-triazin-2-yl]-amino)-stilben-2,2'-disulfonsäure-di-triäthanolammoniumsalz.

Ferner wird feste PNTSA bei der Synthese von optischen Aufhellern anderer Grundstruktur, wie z. B. 2-(4-Styryl-3-sulfophenyl)-2H-naphtho-[1,2-d]triazol (Na-Salz), verwendet. Weitere beträchtliche Mengen an PNTSA werden für die Synthese von anderen optischen Aufhellern sowie von verschiedenen Farbstoffen eingesetzt.

Das ursprüngliche Standardverfahren zur Herstellung von PNTSA bestand in der Sulfonierung von PNT mit 20%igem Oleum (80 Teile $SO_3$, gelöst in 320 Teilen $H_2SO_4$). Nach Beendigung dieser Sulfonierung enthält das Reaktionsgemisch etwa 39% PNTSA und 61% $H_2SO_4$. Die Reaktionsmischung wird dann mit Wasser so weit verdünnt, daß eine 56—70%ige schwefelsaure wäßrige Lösung entsteht, aus der die Hauptmenge an PNTSA auskristallisiert. Wiederaufarbeitung oder Beseitigung der sauren Mutterlauge bedeuten jedoch einen beträchtlichen ökonomischen Nachteil dieses Verfahrens. Bei $H_2SO_4$-Konzentrationen oberhalb etwa 20%, bezogen auf PNTSA, kann die PNTSA-haltige Lösung kaum direkt im DNS-Herstellungsverfahren eingesetzt werden. Außerdem fallen beim vorstehend beschriebenen Verfahren, selbst wenn es so effizient wie möglich durchgeführt wird, ungefähr 1,5 Gewichtsteile eines Schwefelsäure-Effluenten pro 1 Gewichtsteil PNTSA an. Die Sulfonierung von PNT mit Oleum mit einem $SO_3$-Gehalt über 40% läuft nicht ohne teilweise Zerstörung von PNT ab, wodurch PNTSA nur in bescheidener Ausbeute und als schwarzes Produkt erhalten wird. Es wurde auch vorgeschlagen, 65%iges Oleum zu verwenden, wobei PNT vor Zugabe des Oleums mit Sulfonierungsmasse verdünnt wird. Es sind jedoch, wie in allen Oleum-Verfahren, Filtrations- und Kristallisationsschritte nötig, um die unerwünschte Schwefelsäure zu entfernen.

Um die Nachteile des Oleum-Verfahrens zu beseitigen, wurden bereits weitere Sulfonierverfahren entwickelt, in denen gasförmiges $SO_3$ verwendet wird. In der US-Patentschrift 3 840 591 ist ein Verfahren für die Herstellung von PNTSA beschrieben, bei dem geschmolzenes PNT direkt mit einer Mischung aus $SO_3$ und einem Inertgas sulfoniert wird. Das in geringem stöchiometrischem Überschuß verwendete $SO_3$ wird mit einem Inertgas, z. B. Stickstoff, verdünnt, um die Reaktionsgeschwindigkeit, die Reaktionstemperatur und die Menge an Nebenprodukten zu steuern. Es entstehen jedoch komplexe farbige Produkte, die sich im Endprodukt in einem Maße anreichern, das dessen direkte kommerzielle Verwertbarkeit stört. Die nötigen Entfärbungsoperationen erhöhen die Kosten des fertigen Produktes.

In anderen Verfahren mit nahezu vollständiger Umsetzung von PNT zu PNTSA, die ohne Verdünnungs- oder Extraktionsmittel durchgeführt werden, sind die Reaktionstemperaturen (140—150° C) zu nahe an der Zersetzungstemperatur von PNTSA bzw. PNTSA-Anhydrid (ca. 150° C). Eine solche Zersetzung beeinflußt die Sicherheit des Verfahrens nachteilig. Es wurden daher Lösungsmittel vorgeschlagen, die als Verdünner bzw. Lösungsvermittler während der Sulfonierungsreaktion dienen sollten. In Lösungsmittelverfahren zur Sulfonierung von PNT mit $SO_3$ wird entweder ein Überschuß an PNT oder ein organisches Lösungsmittel, wie z. B. Tetrachloräthylen oder Dichloräthan, als Verdünnungsmittel verwendet. Das Lösungsmittel bzw. der Überschuß an PNT dient hauptsächlich auch dazu, die Reaktionsmasse in einem Temperaturbereich von 80—150°C flüssig zu halten. Die in diesem Verfahren schließlich erhaltenen Reaktionslösungen müssen dekantiert, extrahiert und/oder ausgewaschen werden, um nicht umgesetztes PNT zu entfernen oder um das organische Lösungsmittel zurückzugewinnen. Bei Verwendung von organischem Lösungsmittel wird PNT zu nahe 99% umgesetzt. Wird in Überschuß an PNT gearbeitet, kann die Umsetzung etwa 90—95% nicht übersteigen, wenn die Reaktionsmasse flüssig bleiben soll. Aus Gründen der leichten Durchführbarkeit der Reaktion und der Reinheit des Endproduktes ist ein Umsetzungsgrad von etwa 75% bevorzugt.

Die deutsche Offenlegungsschrift 2 916 912 beschreibt eine Niedertemperatur-Sulfonierung im Bereich zwischen 60 und 150°C in geschlossenem Reaktionsgefäß bei Normaldruck unter Verwendung von reinem $SO_3$ als Sulfonierungsreagens. Um das Verfahren vorteilhaft zu gestalten, muß bei Überschuß an PNT gearbeitet werden. Die Abtrennung des PNT-Überschusses bedeutet einen ökonomischen Nachteil dieses Verfahrens, da der PNT-Überschuß praktisch ein teures Reaktionslösungs- und

-verdünnungsmittel darstellt.

Eine Aufgabe der vorliegenden Erfindung besteht darin, PNTSA direkt in einem Schritt in unmittelbar weiterverwendbarer Form mit annehmbaren $H_2SO_4$-Konzentrationen herzustellen. Eine weitere Aufgabe der Erfindung ist es, ein Herstellungsverfahren für PNTSA zu schaffen, das aufwendige und teure Kristallisations-, Filtrations-, Wasch- und Wiederauflösungsschritte am Endprodukt überflüssig macht. Schließlich ist es noch Aufgabe der Erfindung, ein Verfahren bereitzustellen, das PNTSA in einer annehmbaren Farbe, in hoher Reinheit und frei von organischen Lösungsmitteln liefert.

Es wurde überraschenderweise gefunden, daß der Zusatz einer kleinen Menge Schwefelsäure zum zu sulfonierenden geschmolzenen PNT die gestellten Aufgaben löst und daß dadurch die Sulfonierung praktisch vollständig abläuft mit einem guten Umsetzungsgrad (99—100%), in guter Ausbeute (99,3—100%) und zu einem Produkt mit niederem Gardner-Färbestandard führt.

Die Erfindung betrifft daher ein Verfahren zur Herstellung von p-Nitrotoluol-2-sulfonsäure (PNTSA) durch Sulfonierung einer Schmelze von p-Nitrotoluol (PNT) mit gasförmigem $SO_3$ bei erhöhter Temperatur und ist dadurch gekennzeichnet, daß man der Schmelze von PNT 0,08—0,33 Teile Schwefelsäure (in Form von Schwefelsäure selbst oder in Form einer Schwefelsäure enthaltenden oder freisetzenden Substanz) pro Teil PNT zugibt, und diese Mischung mit 95—130% der theoretischen Menge an gasförmigem $SO_3$, das eine Temperatur von 44—180° C hat, sulfoniert, wobei man die Reaktionsmischung bis zur praktisch vollständigen Umsetzung bei einer Temperatur von 75 bis 150° C hält.

Die Reaktionsmischung wird beispielsweise etwa 2 Stunden im letztgenannten Temperaturintervall gehalten, bis die Reaktion beendet ist.

Nach Beendigung der Reaktion enthält die Sulfonierungsmasse beispielsweise etwa 85—88% PNTSA, 0—0,05% PNT, 0,1—1% Sulfon und 9—14% Schwefelsäure, bezogen auf PNTSA. In einer Ausführungsform wird diese Masse isoliert und kann direkt im Herstellungsverfahren für DNS eingesetzt werden. Bevorzugt wird die Sulfonierungsmasse verdünnt, bevor sie im DNS-Verfahren weiterverarbeitet wird.

Prinzipiell kann die PNTSA enthaltende Reaktionsmasse für den weiteren Einsatz mit Wasser verdünnt und dann direkt im DNS-Herstellungsverfahren eingesetzt werden, oder die PNTSA kann als solche isoliert werden. Im Falle der Verdünnung wird eine Lösung angestrebt, die etwa 30—35% PNTSA und 4,5—7% Schwefelsäure enthält.

Die PNTSA kann z. B. auch in Form eines feuchten Filterkuchens gewonnen werden, der weniger als 1% Schwefelsäure, bezogen auf PNTSA, enthält, indem die Sulfonierungsmasse bis zu einem PNTSA-Gehalt von etwa 43—50% mit Wasser verdünnt und kontinuierlich abgekühlt wird, z. B. auf etwa 25—35° C, wobei etwa 25% der PNTSA auskristallisieren. Der durch Filtration gewonnene feuchte Filterkuchen enthält ohne Waschen etwa 1% $H_2SO_4$. Das Filtrat ist immer noch mit PNTSA gesättigt (ca. 35—40%) und es kann direkt im DNS-Herstellungsverfahren eingesetzt werden. Es enthält etwa 18—20% $H_2SO_4$, bezogen auf PNTSA. Der erhaltene Filterkuchen stellt eine praktische Lager- und Handelsform dar. Er kann nach entsprechender Lösung in Wasser ebenfalls direkt im DNS-Herstellungsverfahren eingesetzt werden.

Eine andere Möglichkeit der Isolierung besteht in der Ausfällung von PNTSA aus der Sulfonierungsmasse durch Schwefelsäure. Die Kristallisation erfolgt z. B. bei einer $H_2SO_4$-Konzentration von 60%. Das Filtrat soll aus ökonomischen und ökologischen Gründen recycliert werden.

Da die nach dem erfindungsgemäßen Verfahren erhaltene Sulfonierungsmasse beispielsweise ungefähr 85—88% PNTSA und 9—14% Schwefelsäure enthält, kann sie auch direkt abgezogen und zur Verfestigung stehen gelassen werden, statt sie einer der drei obengenannten Aufarbeitungsoperationen zu unterziehen. Die Sulfonierungsmasse wird bei etwa 70° C fest und bildet bei Raumtemperatur einen spröden, glasartigen, leicht zu mahlenden Festkörper, der nicht hygroskopisch und gut lagerfähig ist. Das gemahlene Pulver kann dann gelöst und im DNS-Herstellungsverfahren eingesetzt werden.

Die Sulfonierungsreaktion verläuft ungefähr nach folgendem Schema:

Hauptreaktion:

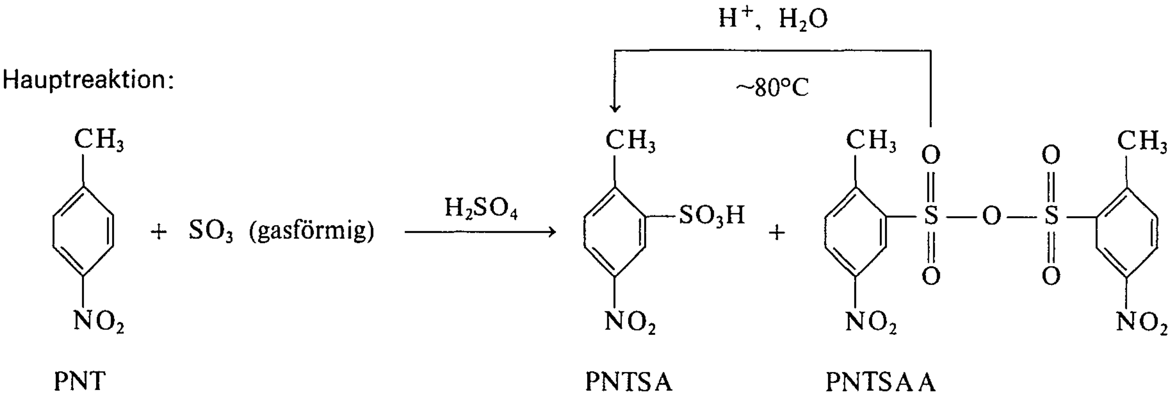


PNT PNTSA PNTSAA

(p-Nitrotoluol-2-sulfonsäure-anhydrid)

Nebenreaktion:

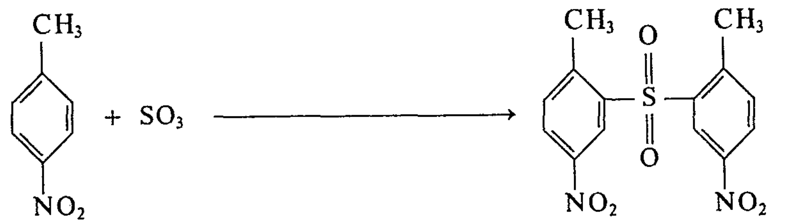

PNT p-Nitrotoluolsulfon (»Sulfon«)

Die eingesetzte Menge an Schwefelsäure in der Reaktionsmischung bewirkt, daß das Sulfon und das Anhydrid in wesentlich geringerem Maß entsteht als in ähnlichen Verfahren. Außerdem hält sie die Reaktionsmasse bis hinunter zu 80° C flüssig. Deswegen erreicht man mit dem erfindungsgemäßen Verfahren PNT-Umsetzungsgrade von mindestens 99% und PNTSA-Ausbeuten von mindestens etwa 99,3%, vor allem, wenn $SO_3$ in stöchiometrischem Überschuß von etwa 105—107% verwendet wird.

Da die geringe Menge an Schwefelsäure, die sich noch in der verdünnten PNTSA-Endlösung befindet, beim folgenden DNS-Herstellungsverfahren verbraucht wird, tritt beim erfindungsgemäßen Verfahren in seiner bevorzugten Ausführungsform (Weiterverwendung der verdünnten Reaktionsmasse zur DNS-Herstellung) kein Effluent auf. Während der Reaktion wird außerordentlich wenig Sulfon gebildet, was einen besonderen Vorteil des Verfahrens darstellt.

Das erfindungsgemäße Verfahren liefert also ein Reaktionsprodukt in folgenden Formen:

a) Als flüssige Sulfonierungsmasse, enthaltend beispielsweise etwa 87% PNTSA und 12—13% $H_2SO_4$, welche ohne weitere Aufarbeitung direkt im DNS-Herstellungsverfahren eingesetzt werden kann.
b) Als feste Sulfonierungsmasse, die ebenfalls beispielsweise etwa 87% PNTSA und 12—13% $H_2SO_4$ enthält.
c) Als verdünnte Lösung, enthaltend z. B. 30—35% PNTSA, die ebenfalls im DNS-Herstellungsverfahren direkt eingesetzt werden kann.
d) Als feuchter PNTSA-Filterkuchen mit weniger als 1% $H_2SO_4$ (bezogen auf PNTSA); das Filtrat, das eine gesättigte PNTSA-Lösung mit etwa 20% $H_2SO_4$ (bezogen auf PNTSA) darstellt, kann ebenfalls direkt im DNS-Herstellungsverfahren eingesetzt werden.

Die Gesamtmenge an Schwefelsäure, die sich aus der dem geschmolzenen PNT zugegebenen Schwefelsäure und dem als Sulfonierungsreagens verbrauchten $SO_3$ zusammensetzt, ist beispielsweise beim bevorzugten $H_2SO_4$/PNT-Verhältnis von 0,142 ungefähr 85%igem Oleum äquivalent. Wenn Oleum mit einer $SO_3$-Konzentration von mehr als 40% mit PNT in Berührung kommt, kommt es zu Karbonisierung. Diese reduziert die Ausbeute, einerseits wegen der dadurch verminderten Menge an PNT und anderseits wegen der ebenfalls eintretenden Karbonisierung und Zersetzung von gebildetem PNTSA, PNTSAA und Sulfon. Bei der erfindungsgemäßen Verfahrensführung tritt dieser Nachteil jedoch nicht auf.

Die dem geschmolzenen PNT zugesetzte Schwefelsäure bzw. die als Schwefelsäurequelle dienende Substanz kann z. B. verdünnte oder 100%ige Schwefelsäure sein; letztere kann aber auch geringe Mengen an $SO_3$ enthalten, um die Reaktion zu starten, nämlich bis zu 30% $SO_3$ (30%iges Oleum). Wenn 96%ige Schwefelsäure eingesetzt wird, wird zuerst $SO_3$ durch die 4% Restwasser absorbiert, wodurch weitere Schwefelsäure entsteht. Wenn an Stelle von Schwefelsäure Oleum (bis zu 30%) eingesetzt wird, muß die Menge an zugeführtem gasförmigem $SO_3$ entsprechend angepaßt werden, da die im Oleum enthaltene $SO_3$-Menge berücksichtigt werden muß. Wenn Oleum verwendet wird, ist 20%iges Oleum bevorzugt.

Als $SO_3$-Quellen können beispielsweise dienen: a) im Handel erhältliches flüssiges $SO_3$, das verdampft und entweder unter die Oberfläche der PNT/$H_2SO_4$-Mischung oder in den oberen Teil des Reaktionsgefäßes eingeleitet wird; b) $SO_3$, das durch Hitze aus mindestens 50%igem Oleum, bevorzugt aus handelsüblichem 65%igem Oleum ausgetrieben wird. Ein Vorteil dieser $SO_3$-Quelle besteht, abgesehen vom günstigeren Preis pro $SO_3$-Einheit, darin, daß nach Austreiben des $SO_3$ 20%iges Oleum zurückbleibt. Letzteres kann direkt an Stelle von $H_2SO_4$ dem geschmolzenen PNT zugegeben werden, wenn, wie oben erwähnt, die $SO_3$-Menge für die Sulfonierung entsprechend angepaßt wird.

Versuche haben ergeben, daß das $H_2SO_4$/PNT-Verhältnis von 0,08 bis 0,33 betragen kann. Der PNT-Umsetzungsgrad beträgt dann etwa 99,1 bis 100%, die Ausbeute etwa 99,3 bis 100%. Das bevorzugte $H_2SO_4$/PNT-Verhältnis beträgt 0,115 bis 0,145, insbesondere etwa 0,142. In diesem Bereich ist der $H_2SO_4$-Gehalt im Endprodukt (Sulfonierungsmasse) etwa 11 bis 15%, bezogen auf PNTSA. Diese Sulfonierungsmasse kann direkt als solche, oder vorzugsweise nach einfachem Verdünnen (keine Kristallisation und Filtration), im DNS-Herstellungsverfahren verwendet werden. Bei dem genannten

bevorzugten $H_2SO_4$/PNT-Verhältnis beträgt der PNT-Umsetzungsgrad 99,8 bis 100%, die Ausbeute an PNTSA mehr als 99,3% und der Gardner-Färbestandard 8—9,5. Für den direkten Einsatz im DNS-Herstellungsverfahren und für die meisten übrigen Erfordernisse sind diese Spezifikationen völlig ausreichend.

Bei der Untersuchung des Verlaufes der Sulfonierungsreaktion mit analytischen Methoden wurde gefunden, daß ungefähr 3—4% PNTSAA (Anhydrid) gebildet wurden und daß letzteres praktisch vollständig in PNTSA übergeführt wird, wenn die Reaktionsmasse nach der Sulfonierung für etwa $^1/_2$ bis 3 Stunden auf einer Temperatur über 80° C gehalten wird. Wenn Anhydrid noch nach dieser Zeit vorhanden ist, wird es während der Verdünnung der Reaktionsmasse schnell hydrolysiert, wenn die Temperatur über 80° C beträgt.

Das eingeleitete $SO_3$-Gas hat eine Temperatur zwischen 44 und 180°C. Praktisch vollständige Umsetzung wird erreicht, wenn die Gastemperatur oberhalb 100°C liegt. Wenn $SO_3$ aus 65%igem Oleum ausgetrieben wird, soll der $SO_3$-»Stripper« bei 150 bis 180° C arbeiten, um 20%iges Oleum und $SO_3$-Gas mit hinreichendem Druck zu erzeugen, um letzteres unter die Oberfläche des Reaktionsgemisches einleiten zu können. Im allgemeinen beeinflußt die Temperatur des $SO_3$-Gases innerhalb des Bereiches von 44—180° C den PNT-Umsetzungsgrad, die Ausbeute an PNTSA, den Schwefelsäuregehalt im Endprodukt und den Färbestandard der Lösung nicht. Jede Gastemperatur von 100 bis 150° C ist jedoch bevorzugt.

Im allgemeinen wird das $SO_3$ bevorzugt unter die Oberfläche der Reaktionsmasse eingeleitet, es kann aber auch in den Raum des Reaktionsgefäßes oberhalb der Reaktionsmasse eingeführt werden. Auch im letzteren Fall löst sich das $SO_3$ schnell in der Sulfonierungsmasse und es wird kein Entweichen durch den Kühler des Reaktionsgefäßes beobachtet.

Um eine möglichst vollständige Umsetzung des PNT zu erreichen (<0,15% PNT im Endprodukt, bezogen auf PNTSA), wird vorzugsweise ein leichter Überschuß an $SO_3$ verwendet. Vollständige Umsetzung wird erreicht, wenn die eingesetzte Menge an $SO_3$ über 103%, z. B. 103—110%, vorzugsweise bei etwa 107% der theoretischen Menge liegt.

Die Sulfonierungsreaktion wird bei Temperaturen von 75—150° C durchgeführt (d. h. die Temperatur der Reaktionsmasse wird in diesem Bereich gehalten). Bei den tieferen Temperaturen dieses Bereiches, z. B. unterhalb etwa 90° C, verläuft die Reaktion langsam unter bevorzugter Bildung von PNTSAA (Anhydrid). Dies bedeutet jedoch keinen Verlust, das PNTSAA in der Reaktionsmasse bei über 80° C zu PNTSA umgewandelt wird. Vorteilhaft ist daher der Bereich zwischen etwa 95 und 130° C, besonders bevorzugt jener von 105 bis 115° C. Im gesamten Bereich ist jedenfalls gewährleistet, daß PNT vollständig geschmolzen ist.

Nach Einleiten des $SO_3$-Gases wird die Reaktionsmischung vorzugsweise noch auf der Reaktionstemperatur gehalten, bis PNT vollständig verbraucht ist. In der Regel ist dies nach etwa 15 bis 20 Minuten der Fall, jedoch kann in der Praxis die Mischung noch etwa $^1/_2$ bis 2 Stunden auf Reaktionstemperatur belassen werden. Nach Beendigung der Reaktion wird die Reaktionsmischung abgekühlt und PNTSA wird auf irgendeine weiter oben beschriebene Form isoliert bzw. die Mischung selbst wird in eine direkt weiterverwendbare Form gebracht.

Der Hauptvorteil des erfindungsgemäßen Verfahrens besteht darin, daß es PNTSA in einer direkt weiterverwendbaren Form praktisch ohne Nebenprodukte oder Rückstände, die weitere Reinigung, Aufarbeitung, Recyclierung oder Abfallbeseitigung erfordern, liefert.

Das in sehr geringen Mengen im Endprodukt vorhandene Sulfon braucht vor der Weiterverarbeitung nicht entfernt zu werden, da es in dieser geringen Menge im DNS-Herstellungsverfahren nicht stört.

Das erfindungsgemäße Verfahren kann beispielsweise diskontinuierlich (»batchwise«) ausgeführt werden. In der Praxis wird es jedoch mit Vorteil kontinuierlich betrieben.

Das DNS-Herstellungsverfahren, in dem das erfindungsgemäß herstellbare PNTSA eingesetzt werden kann, verläuft beispielsweise nach folgendem Schema:

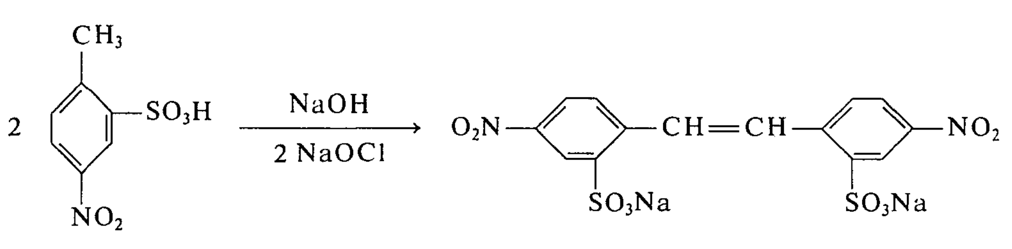


PNTSA DNS

Für die Herstellung der eingangs erwähnten optischen Aufheller wird die DNS zur 4,4'-Diaminostilben-2,2'-disulfonsäure reduziert.

PNTSA, die für die Herstellung von optischen Aufhellern und ähnliche Anwendungen geeignet ist, sollte im wesentlichen die folgenden Spezifikationen erfüllen:

PNT-Gehalt: höchstens 0,15%
Sulfon-Gehalt: höchstens 0,9%
Gehalt an freier Schwefelsäure: höchstens 22—25%
Färbung einer 20%igen wäßrigen Lösung (Gardner-Färbestandard): höchstens 12.
Die Prozentangaben beziehen sich auf PNTSA.

In den folgenden Beispielen werden besonders bevorzugte Ausführungsformen des erfindungsgemäßen Verfahrens beschrieben. Es wird jedoch betont, daß die Erfindung nicht auf die Beispiele beschränkt ist und daß das erfindungsgemäße Verfahren innerhalb der oben angegebenen Parameter ausführbar ist.

Sofern nichts anderes angegeben ist, beziehen sich in der gesamten Beschreibung und in den Patentansprüchen Teile- und Prozentangaben auf das Gewicht.

## Beispiel 1

a) 205,5 g (1,5 Mol) PNT und 20 g 96%ige $H_2SO_4$ werden in einem Glasreaktor auf über 80° C erhitzt, bis die Mischung geschmolzen ist. Das Einleitrohr wird mit Stickstoff gespült, um es frei von PNT/$H_2SO_4$ zu halten, bis $SO_3$ eingeleitet wird. $SO_3$-Gas (erhalten durch Verdampfen von flüssigem $SO_3$) wird dann unter die Oberfläche der geschmolzenen Mischung eingeleitet. Die Reaktion ist schwach exotherm. Ungefähr 72 ml flüssiges $SO_2$, entsprechend 133,2 g (1,67 Mol) $SO_3$ werden im Verlauf einer Stunde eingeleitet. Die Temperatur der Reaktionsmasse wird bis zur Beendigung der $SO_3$-Einleitung durch Kühlung bei 100 bis 105° C gehalten. Danach wird die Reaktionsmasse für 30—60 Minuten zwischen 100 und 115° C gehalten, bis das PNT vollständig verbraucht ist. Ein einfacher Test für die Vollständigkeit der Reaktion besteht darin, daß man eine kleine Menge der Reaktionsmasse in Wasser gibt. Falls noch PNT vorhanden ist, wird dessen charakteristischer Geruch in der warmen Lösung wahrgenommen. Eine gas- oder flüssigchromatographische Untersuchung kann ebenfalls vorgenommen werden. Die Reaktion wird als vollständig angesehen, wenn kein Geruch nach PNT mehr wahrnehmbar ist oder wenn der mittels Gas- oder Flüssigchromatographie festgestellte PNT-Gehalt höchstens 0,1% beträgt. Das vollständig sulfonierte Produkt, das 85—88% PNTSA und $H_2SO_4$ enthält, ist dann für die weitere Verwendung bereit.

b) Die Sulfonierungsmasse wird auf ungefähr 80° C abgekühlt und dann mit 600 ml kaltem Wasser verdünnt. Die Zugabe von Wasser bewirkt eine exotherme Reaktion, die anfänglich eine Kühlung erfordert, um Überhitzung zu vermeiden. Nach beendeter Wasserzugabe wird die verdünnte Lösung für etwa 30 Minuten auf 80—90° C erhitzt, um vollständige Lösung der Reaktionsmasse und die vollständige Umwandlung von möglicherweise gebildetem PNTSAA in PNTSA sicherzustellen. Die Lösung wird dann abgekühlt und kann im DNS-Herstellungsverfahren eingesetzt werden. Der Umsetzungsgrad, bezogen auf PNT, beträgt 100%. Die Ausbeute an PNTSA ist 99,3%. Die verdünnte Lösung enthält 33,3% PNTSA und ungefähr 4,5% $H_2SO_4$. Der Gardner-Färbestandard der Lösung (eingestellt auf 20% PNTSA) ist 9,5.

## Beispiel 2

Das Verfahren nach Beispiel 1 a) und b) wird wiederholt, jedoch werden an Stelle der Schwefelsäure 38,36 g 20%iges Oleum dem geschmolzenen PNT zugesetzt. Außerdem wird die $SO_3$-Menge auf etwa 66 ml, entsprechend 116,64 g $SO_3$ (105% der stöchiometrischen Menge) eingestellt. Die schließlich erhaltene verdünnte Lösung enthält 33,56% PNTSA und 4,6% $H_2SO_4$ und weist einen Gardner-Färbestandard von 11 auf. Nach Filtration findet man 0,19% unlösliches Sulfon.

## Beispiel 3

Das Verfahren nach Beispiel 1 a) wird wiederholt. Danach wird die erhaltene Sulfonierungsmasse in eine Teflonschale gegossen und abkühlen gelassen. Man erhält bei etwa 45° C einen dunklen Festkörper, der nach etwa 24 Stunden heller wird und der nach einer Mahlung 369 g eines hellbraunen Pulvers ergibt, das 81,5% PNTSA und 12,4% Schwefelsäure enthält.

## Beispiel 4

### Kontinuierliches Verfahren

a) Zwei Glasreaktoren werden miteinander verbunden. Der erste, in den PNT, Oleum und $SO_3$-Gas eingeleitet werden, hat ein Volumen von 150 ml, der zweite ein solches von 400 ml. In beiden Reaktoren wird eine Temperatur von 110—120° C aufrechterhalten. In den ersten Reaktor wird PNT mit einer Geschwindigkeit von 5 g/min, 10,91%iges Oleum mit einer Geschwindigkeit von 0,869 g/min und $SO_3$ mit einer Geschwindigkeit von 2,96 g/min eindosiert. Die Reaktionsmischung wird dann in den zweiten Reaktor gepumpt. Die Verweilzeit der Reaktionsmischung beträgt 27 Minuten im ersten und 70 Minuten im zweiten Reaktor.

b) Die Schmelze, die aus dem zweiten Reaktor abgezogen wird, ist den gemäß Schritt a) in den Beispielen 1 und 2 erhaltenen Produkten äquivalent. Sie wird vorzugsweise in ein 320-ml-Verdünnungsgefäß geleitet und wird in diesem dritten Gefäß (Reaktor) mit Wasser verdünnt, wobei die Temperatur auf etwa 90—102° C gehalten wird. Das Wasser wird in diesen dritten Reaktor mit einer Geschwindigkeit von 14,6 g/min eindosiert und die Verweilzeit der verdünnten Lösung in diesem Gefäß beträgt 16 Minuten. Nach Einstellung eines stabilen Reaktionsablaufes werden dem ersten Reaktor innerhalb 90 Minuten etwa 3,285 Mol PNT zugeführt, die 3,149 Mol PNTSA und 0,014 Mol nicht umgesetztes PNT ergeben, was einem Umsetzungsgrad von 99,6% entspricht. Die Menge an gebildetem Sulfon beträgt 0,4%; die $H_2SO_4$-Konzentration beträgt 15,6%, bezogen auf die Gesamtmenge an PNTSA (in der Reaktionsmasse), und im Mittel 4,8% in der verdünnten Lösung. Der Gardner-Färbestandard liegt zwischen 11,5 und 12.

## Beispiel 5

Die nach den Beispielen 1 a), 2 a) und 4 a) erhaltene Sulfonierungsmasse oder das wieder geschmolzene, nach Beispiel 3 erhaltene feste Produkt wird mit Wasser auf eine PNTSA-Konzentration von etwa 50% verdünnt. In einer üblichen Kristallisationsapparatur wird die Lösung gekühlt und etwa 25—30% der PNTSA kristallisieren aus und werden als feuchter Filterkuchen isoliert. Dieser enthält etwa 88% PNTSA bzw. 98% PNTSA · 2 $H_2O$ und etwa 1% Schwefelsäure. Das Filtrat erfüllt die Bedingungen für einen direkten Einsatz im DNS-Herstellungsverfahren.

## Beispiel 6

### Herstellung von DNS

In einem 5-Halskolben aus Glas (Inhalt: 5 Liter) mit Untenauslauf, einem regelbaren Schnellrührer, Thermometer, Heizmantel, Kühler, Redoxpotential-Meßgerät mit Elektroden, pH-Elektroden und zwei Tropftrichteranschlüssen, einer mit einem 500-ml-Tropftrichter, der andere mit einem 250-ml-Tropftrichter mit Heizmantel bestückt, werden 2195 g Wasser und 256 g 50%ige wäßrige Natronlauge vorgelegt. Der Rührer wird in Gang gesetzt und der Inhalt auf 85—86° C erhitzt. Das Redoxpotential soll bei 85° C etwa 650—800 mv betragen. 700 g Chlorbleichlösung (15—17% aktives Chlor) werden in den 500-ml-Tropftrichter eingefüllt. 248,1 g einer z. B. gemäß einem der vorhergehenden Beispiele erhaltenen, bei 80—100°C geschmolzenen Sulfonierungsmasse, enthaltend etwa 85% PNTSA, 0,1% PNT, 0,41% Sulfon und 12% Schwefelsäure werden in den 250-ml-Tropftrichter eingefüllt.

Wenn der Kolbeninhalt 85°C erreicht hat, werden 4—5 ml der Bleichlösung zugegeben, bis ein Redoxpotential von 1000—1050 mv erreicht ist. Die PNTSA enthaltende Schmelze wird nun mit einer Geschwindigkeit von etwa 15—20 ml/min zugetropft, wobei die gesamte Schmelze in etwa 15—20 Minuten verbraucht ist. Während des Zutropfens fällt das Redoxpotential auf etwa 910 mv. Dann wird Bleichlösung mit einer solchen Geschwindigkeit zugetropft, daß das Redoxpotential auf 900—910 mv bleibt, bis die PNTSA-Zugabe beendet ist. Danach wird der 250-ml-Tropftrichter durch einen solchen ersetzt, der 70—90 g 98%ige $H_2SO_4$ enthält. Nach 50 Minuten Reaktionszeit (gerechnet vom Beginn der PNTSA-Zugabe) wird auf 76°C abgekühlt, nach Erreichen dieser Temperatur die Zugabe von Bleichlösung eingestellt und mit 70—90 g 98%iger $H_2SO_4$ auf einen pH-Wert von 5,5—6 neutralisiert. Die Reaktionsmischung wird nun in ein 4-Liter-Becherglas gegossen, auf 20°C abgekühlt und 30 Minuten bei 20° C gehalten. Das ausgefallene Produkt (DNS) wird abfiltriert und bei 60° C getrocknet. Der Umsetzungsgrad, bezogen auf PNTSA, beträgt 97 ± 1,7%, die Totalausbeute 78,9 ± 2,3% mit einem Gehalt an reiner DNS von mehr als 95%.

## Patentansprüche

1. Verfahren zur Herstellung von p-Nitrotoluol-2-sulfonsäure durch Sulfonierung einer Schmelze von p-Nitrotoluol mit gasförmigem $SO_3$ bei erhöhter Temperatur, dadurch gekennzeichnet, daß man der Schmelze von p-Nitrotoluol 0,08—0,33 Teile Schwefelsäure, in Form von Schwefelsäure selbst oder in Form einer Schwefelsäure enthaltenden oder freisetzenden Substanz, pro Teil p-Nitrotoluol zugibt und diese Mischung mit 95—130% der theoretischen Menge an gasförmigem $SO_3$, das eine Temperatur von 44—180°C hat, sulfoniert, wobei man die Reaktionsmischung bis zur praktisch vollständigen Umsetzung bei einer Temperatur von 75 bis 150°C hält.

2. Verfahren nach Anspruch 1, dadurch gekennzeichnet, daß man die erhaltene Sulfonierungsmasse direkt weiter zu 4,4'-Dinitrostilben-2,2'-disulfonsäure durch Kondensation der p-Nitrotoluol-2-sulfonsäure in Gegenwart von wäßrigem Alkali und einem Oxidationsmittel umsetzt.

3. Verfahren nach Anspruch 1, dadurch gekennzeichnet, daß man die erhaltene Sulfonierungsmasse mit Wasser so verdünnt, daß eine wäßrige schwefelsaure Lösung von p-Nitrotoluol-2-sulfonsäure entsteht, die man direkt für die Herstellung von 4,4'-Dinitrostilben-2,2'-disulfonsäure einsetzen kann.

4. Verfahren nach Anspruch 1, dadurch gekennzeichnet, daß man die erhaltene Sulfonierungsmasse mit Wasser zu einer ungefähr 43 bis 50%igen p-Nitrotoluol-2-sulfonsäurelösung verdünnt, letztere kühlt und die ausgefallenen p-Nitrotoluol-2-sulfonsäurekristalle, die weniger als 1% Schwefelsäure enthalten, abfiltriert.

5. Verfahren nach Anspruch 1, dadurch gekennzeichnet, daß man die erhaltene Sulfonierungsmasse abkühlen läßt und gegebenenfalls die entstandene brüchige feste Masse zu einem Pulver vermahlt.

6. Verfahren nach Anspruch 1, dadurch gekennzeichnet, daß man 0,115 bis 0,145, insbesondere 0,142 Teile Schwefelsäure pro Teil p-Nitrotoluol einsetzt.

7. Verfahren nach Anspruch 1 oder 6, dadurch gekennzeichnet, daß man die Schwefelsäure in Form von verdünnter oder 100%iger Schwefelsäure einsetzt.

8. Verfahren nach Anspruch 1 oder 6, dadurch gekennzeichnet, daß man die Schwefelsäure in Form von bis zu 30%igem Oleum, vorzugsweise 20%igem Oleum, einsetzt.

9. Verfahren nach Anspruch 1, dadurch gekennzeichnet, daß mit 103 bis 110% der für die Sulfonierung von p-Nitrotoluol benötigten Menge an $SO_3$ sulfoniert wird.

10. Verfahren nach Anspruch 1, dadurch gekennzeichnet, daß man die Temperatur der Reaktionsmasse während der Sulfonierung bei 95 bis 130°C, vorzugsweise 105 bis 115°C hält.

11. Verfahren nach Anspruch 1, dadurch gekennzeichnet, daß man die Temperatur nach Beendigung der $SO_3$-Einleitung zur Vervollständigung der Umsetzung bei über 80°C hält.

12. Verfahren nach Anspruch 4, dadurch gekennzeichnet, daß man das Filtrat, falls erforderlich, durch weitere Verdünnung auf eine solche p-Nitrotoluol-2-sulfonsäure- und Schwefelsäurekonzentration einstellt, daß es direkt im Herstellungsverfahren für 4,4'-Dinitrostilben-2,2'-disulfonsäure eingesetzt werden kann.

13. Verfahren nach Anspruch 1, dadurch gekennzeichnet, daß man das Verfahren kontinuierlich durchführt.

## Claims

1. A process for the production of p-nitrotoluene-2-sulfonic acid by sulfonating molten p-nitrotoluene with gaseous $SO_3$ at elevated temperature, which process comprises mixing molten p-nitrotoluene with 0.08—0.33 parts of sulfuric acid in the form of sulfuric acid itself or in the form of a source of sulfuric acid per part of p-nitrotoluene, sulfonating the mixture with 95—130% of the theoretical amount of gaseous $SO_3$ which has a temperature in the range from 44—180°C and holding the reaction mixture at a temperature in the range from 75—150°C for a time sufficient for substantial completion of the reaction.

2. A process according to claim 1, which comprises directly further reacting the sulfonation mass to 4,4'-dinitrostilbene-2,2'-disulfonic acid by condensation of the p-nitrotoluene-2-sulfonic acid in the presence of aqueous alkali and an oxidant.

3. A process according to claim 1, which comprises diluting the sulfonation mass with sufficient water to yield an aqueous solution of p-nitrotoluene-2-sulfonic acid in sulfuric acid, which solution can be used direct for the preparation of 4,4'-dinitrostilbene-2,2'-disulfonic acid.

4. A process according to claim 1, which comprises diluting the sulfonation mass with water to about a 43—50% concentration of p-nitrotoluene-2-sulfonic acid, cooling said solution and isolating the precipitated crystals of p-nitrotoluene-2-sulfonic acid containing less than 1% of sulfuric acid.

5. A process according to claim 1, which comprises cooling the sulfonation mass and, if desired, grinding the resultant brittle solid mass to a powder.

6. A process according to claim 1, which comprises using 0.115 to 0.145, preferably 0.142, parts of sulfuric acid per part of p-nitrotoluene.

7. A process according to either claim 1 or claim 6, which comprises using the sulfuric acid in the form of dilute or 100% sulfuric acid.

8. A process according to either claim 1 or claim 6, which comprises using the sulfuric acid in the form of up to 30% oleum, preferably up to 20% oleum.

9. A process according to claim 1, which comprises carrying out the sulfonation with 103—110% of the amount of $SO_3$ required for the sulfonation of the p-nitrotoluene.

10. A process according to claim 1, which comprises holding the reaction mass at a temperature in the range from 95—130° C, preferably from 105—115° C, during the sulfonation.

11. A process according to claim 1, which comprises holding the temperature in the range above 80° C to bring the reaction to completion, after introducing $SO_3$.

12. A process according to claim 4, wherein the filtrate, if necessary, is adjusted by further dilution to a concentration of p-nitrotoluene-2-sulfonic acid and sulfuric acid such that said filtrate can be used direct in the preparation of 4,4′-dinitrostilbene-2,2′-disulfonic acid.

13. A process according to claim 1, wherein said process is carried out continuously.

**Revendications**

1. Procédé pour la préparation de l'acide p-nitrotoluène-2-sulfonique par sulfonation d'une masse fondue de p-nitrotoluène avec $SO_3$ gazeux à température élevée, caractérisé par le fait qu'on ajoute à la masse fondue de p-nitrotoluène, 0,08—0,33 parties d'acide sulfurique sous forme d'acide sulfurique lui-même ou sous forme d'une substance contenant ou libérant de l'acide sulfurique, par partie de p-nitrotoluène, et qu'on sulfone ce mélange avec par 95 à 130% de la quantité théorique de $SO_3$ gazeux qui est à une température de 44—180° C, et qu'on maintient le mélange réactionnel, jusqu'à ce que la réaction soit pratiquement terminée, à une température de 75—150° C.

2. Procédé selon la revendication 1, caractérisé par le fait qu'on transforme directement ensuite la masse de sulfonation obtenue en l'acide 4,4′-dinitrostilbène-2,2′-disulfonique par condensation de l'acide p-nitrotoluène-2-sulfonique en présence de produits alcalins aqueux et d'un oxydant.

3. Procédé selon la revendication 1, caractérisé par le fait qu'on dilue la masse de sulfonation obtenue avec de l'eau de façon à ce qu'il se forme une solution sulfurique aqueuse d'acide p-nitrotoluène-2-sulfonique, qu'on peut utiliser directement pour la préparation de l'acide 4,4′-dinitrostilbène-2,2′-disulfonique.

4. Procédé selon la revendication 1, caractérisé par le fait qu'on dilue la masse de sulfonation obtenue avec de l'eau pour avoir une solution d'environ 43—50% d'acide p-nitrotoluène-2-sulfonique, qu'on refroidit cette dernière, qu'on sépare par filtration les cristaux d'acide p-nitrotoluène-2-sulfonique précipité qui contiennent moins de 1% d'acide sulfurique.

5. Procédé selon la revendication 1, caractérisé par le fait qu'on laisse refroidir la masse de sulfonation obtenue et qu'on broie éventuellement la masse solide fragile formée pour avoir une poudre.

6. Procédé selon la revendication 1, caractérisé par le fait qu'on utilise 0,115 à 0,145, en particulier 0,142 partie d'acide sulfurique par partie de p-nitrotoluène.

7. Procédé selon la revendication 1 ou 6, caractérisé par le fait qu'on utilise l'acide sulfurique sous forme d'acide sulfurique étendu ou d'acide sulfurique à 100%.

8. Procédé selon la revendication 1 ou 6, caractérisé par le fait qu'on utilise l'acide sulfurique sous forme d'oléum jusqu'à 30%, de préférence d'oléum à 20%.

9. Procédé selon la revendication 1, caractérisé par le fait qu'on sulfone avec 103 à 110% de la quantité de $SO_3$ nécessaire pour la sulfonation du p-nitrotoluène.

10. Procédé selon la revendication 1, caractérisé par le fait qu'on maintient la température de la masse réctionnelle pendant la sulfonation à 95—130° C, de préférence à 105—115° C.

11. Procédé selon la revendication 1, caractérisé par le fait qu'on maintient la température une fois l'introduction de $SO_3$ terminée au-dessus de 80° C pour compléter la réaction.

12. Procédé selon la revendication 4, caractérisé par le fait qu'on règle le filtrat, si nécessaire, en le diluant encore à une concentration d'acide p-nitrotoluène-2-sulfonique et à une concentration d'acide sulfurique telles, qu'il peut être utilisé directement dans la préparation de l'acide 4,4′-dinitrostilbène-2,2′-disulfonique.

13. Procédé selon la revendication 1, caractérisé par le fait qu'on effectue le procédé en continu.